(19) Europäisches Patentamt
European Patent Office
Office européen des brevets

(11) Veröffentlichungsnummer: 0 009 126
B1

(12) **EUROPÄISCHE PATENTSCHRIFT**

(45) Veröffentlichungstag der Patentschrift: 13.05.81

(21) Anmeldenummer: 79103038.0

(22) Anmeldetag: 20.08.79

(51) Int. Cl.³: C 07 C 43/23, C 08 G 65/28, D 06 P 1/613

# (54) 1,3-Bisarylglycerinäther und deren Verwendung.

(30) Priorität: 11.09.78 DE 2839463

(43) Veröffentlichungstag der Anmeldung: 02.04.80 Patentblatt 80/7

(45) Bekanntmachung des Hinweises auf die Patenterteilung: 13.05.81 Patentblatt 81/19

(84) Benannte Vertragsstaaten: AT BE CH DE FR GB IT NL SE

(56) Entgegenhaltungen:
DE - A - 2 351 117
FR - A - 2 306 230
FR - A - 2 372 269
CHEMICAL ABSTRACTS, Vol. 86, 1977 Seite 513, Zusammenfassung Nr. 171004j, Columbus, Ohio, USA
Sh. MAMEDOV et al.: "Study of some chemical reactions of 1,3-bis (2,4-diclorophenoxy)-2-propanol"
JAPANESE PATENTS GAZETTE, Woche X18, 13-9-1974, Derwent Publications, London, GB

(73) Patentinhaber: BASF Aktiengesellschaft
Carl-Bosch-Strasse 38
D-6700 Ludwigshafen (DE)

(72) Erfinder: Krapf, Heinz, Dr. Dipl.-Chem.
In der Schleit
D-6718 Grünstadt (DE)
Erfinder: Oppenlaender, Knut, Dr. Dipl.-Chem.
Otto-Dill-Strasse 23
D-6700 Ludwigshafen (DE)
Erfinder: Uhl, Guenter
Pfrimmanlage 55
D-6520 Worms (DE)

EP 0 009 126 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 1,3-Bisarylglycerinäther und deren Verwendung

Die Erfindung betrifft alkoxylierte 1,3-Bisarylglycerinäther und deren Verwendung als Emulgatoren.

Aus der DE—OS 21 39 447 und der DE—OS 21 39 448 sind alkoxylierte 1,3-Glycerinäther bekannt, bei denen sich mindestens ein Ätherrest von einer Alkylgruppe ableitet. Es handelt sich bei diesen Substanzen um oberflächenaktive Verbindungen.

Außerdem sind 1,3-Bisarylglycerinäther bekannt, vgl. J. Med. Chem. *19* (1976) 2 22—229. In dieser Literaturstelle wird die Oxidation der 1,3-Bisarylglycerinäther zu 1,3-Bisaryl-2-propanonen beschrieben.

Die DE—PS 2 128 904 betrifft ein Verfahren zum Bedrucken von Fasermaterialien mit Druckpasten, die auf Öl-in-Wasser-Emulsionen, Pigmentfarbstoffen, Verdickungsmitteln, Pigmentbindern und Emulgatoren vom Typ oxalkylierter Phenolderivate beruhen. Als Emulgator werden 12- bis 16-fach oxäthylierte Di-($\alpha$-phenyläthyl)-phenole verwendet. Diese Verbindungen sind zwar sehr gute Emulgatoren, jedoch gewährleisten sie nicht in allen Fällen eine Reproduzierbarkeit des Druckausfalls.

Aus Chem. Abstr. Vol. 86 (1977), Seite 513, Nr. 171 004 j, sind Reaktionen von 1,3-Bis(2,4-dichlorphenoxy)-2-propanol mit Chlormethyläthern, Carbonsäuren bzw. Acrylnitril bekannt. Die Reaktionsprodukte sind jedoch keine Emulgatoren.

Aus der JP-Anmeldung Nr. 51 032 890 ist ein Verfahren zum Bedrucken von synthetischen Fasermaterialien mit wasserunlöslichen Farbstoffen bekannt, bei dem man eine bessere Fixierung der Farbstoffe auf der Faser erzielt, indem man u.a. auch bestimmte Bis-arylverbindungen als Färbereihilfsmittel einsetzt. Unter den genannten Bis-arylverbindungen sind jedoch keine 1,3-Bisarylglycerinäther. Die aus dieser Literaturstelle bekannten Verbindungen sind ebenfalls keine Emulgatoren.

Aufgabe der Erfindung ist es, Stoffe mit hoher Emulgierwirkung zur Verfügung zu stellen, die bei Verwendung in Drückpasten, die auf Öl-in-Wasser-Emulsionen beruhen, eine gute Reproduzierbarkeit des Druckausfalls gewährleisten.

Die Aufgabe wird erfindungsgemäß gelöst mit alkoxylierten 1,3-Bisarylglycerinäther der Formel

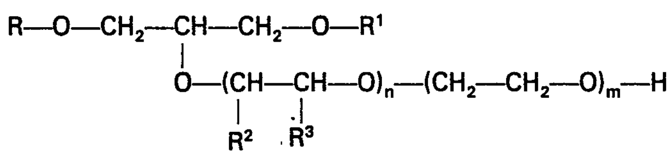

(I)

in der

R, $R^1$ =

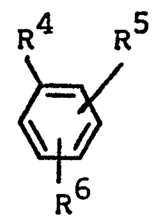

, $\alpha$-Naphthyl oder $\beta$-Naphthyl und

$R^4$, $R^5$, $R^6$ = —H, eine $C_1$- bis $C_{12}$-Alkylgruppe oder

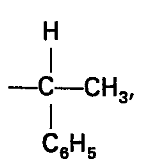

$R^2 = -CH_3, -C_2H_5,$
$R^3 = -H, -CH_3,$
n = 0 bis 10 und
m = 8 bis 50

bedeuten. Die neuen Stoffe sind wasserlöslich und werden als Öl-in-Wasser-Emulgatoren verwendet, vorzugsweise zur Herstellung von Druckpasten.

Die erfindungsgemäßen Stoffe werden hergestellt, indem man 2 Mol Phenol oder eines Phenolderivats mit 1 Mol Alkali umsetzt, das Phenol/Phenolat-Gemisch anschließend mit 1 Mol Epichlorhydrin zur Reaktion bringt und das Reaktionsgemisch nach dem Abtrennen des ausgefallenen Alkalisalzes einer Alkoxylierung unterwirft.

Für die Umsetzung mit Epichlorhydrin geeignete Phenole sind beispielsweise das Phenol selbst $C_1$- bis $C_{12}$-alkylsubstituierte Phenole, z.B. Kresole, Xylenole, p-tert.-Butylphenol, 2,4-Di-tert.-butylphenol, Octylphenol, Nonylphenol, $\alpha$-Phenyläthylphenol, Bis-($\alpha$-phenyläthyl)-phenol, tris-($\alpha$-Phenyläthyl)-phenol, $\alpha$-Naphthol und $\beta$-Naphthol. Verwendet man bei der Umsetzung des Epichlorhydrins mit dem Phenol/Phenolat-Gemisch ein einziges Phenol, so ist in Formel I der Substituent R gleich dem Substituenten $R^1$. Bei der Herstellung der Glycerinäther können Isomerengemische von Phenolen sowie auch unterschiedliche Phenole verwendet werden. In diesen Fällen haben die Substituenten R und $R^1$ verschiedene Bedeutung. Vorzugsweise verwendet man als Phenol für die Herstellung der Glycerinäther Xylenole, z.B. 2,4-Xylenol, 2,5-Xylenol, 2,3-Xylenol, 3,4-Xylenol und 3,5-Xylenol.

Die Herstellung der Glycerinäther kann in Abwesenheit eines Lösungsmittels vorgenommen werden, indem man das Phenol/Phenolat-Gemisch und Epichlorhydrin erhitzt. Die Reaktion erfolgt hier in der Schmelze. Man kann jedoch auch inerte Verdünnungsmittel wie Dioxan, Benzol oder Toluol oder Alkohole wie Isopropanol, Isobutanol und Isoamylalkohol verwenden oder auch die Reaktion in Wasser als Lösungsmittel durchführen. Für den Fall, daß in Formel I R und $R^1$ verschiedene Substituenten bedeuten, setzt man zunächst 1 Mol eines Phenolats mit 1 Mol Epichlorhydrin um und isoliert den zunächst entstehenden Arylglycidyläther, der dann mit einem weiteren Mol eines Phenols, das von dem ersten Phenol verschieden ist, unter Bortrifluoridkatalyse zum 1,3-Bisarylglycerinäther umgesetzt wird.

Die so hergestellten 1,3-Bisarylglycerinäther enthalten noch eine freie OH-Gruppe, die der Alkoxylierung zugänglich ist. Die Alkoxylierungsreaktion wird in bekannter Weise durchgeführt, indem man den OH-gruppenhaltigen 1,3-Bisarylglycerinäther mit Alkylenoxiden zur Reaktion bringt. Als Alkylenoxid kommt vorzugsweise Äthylenoxid in Betracht. Pro Mol 1,3-Bisarylglycerinäther verwendet man 8 bis 50 Mol, vorzugsweise 14 bis 30 Mol Äthylenoxid. Wirksame Emulgatoren erhält man auch dann, wenn man die 1,3-Bisarylglycerinäther, hergestellt aus 2 Mol eines Phenols und 1 Mol Epichlorhydrin, zunächst mit Propylenoxid oder Butylenoxid reagieren läßt und das Reaktionsprodukt anschließend mit 8 bis 50 Mol Äthylenoxid umsetzt. Propylenoxid und die isomeren Butylenoxide werden, bezogen auf den Hydroxylgruppen enthaltenden 1,3-Bisarylglycerinäther, bis zu einer 10-fachen molaren Menge eingesetzt. In diesem Fall bedeutet n in Formel I gleich 10. Sofern man bei der Alkoxylierung Propylenoxid und Butylenoxide oder Mischungen aus Propylenoxid und Butylenoxid verwendet, liegt der Gehalt an Äthylenoxid-Einheiten in den alkoxylierten 1,3-Bisarylglycerinäthern vorzugsweise in dem Bereich von 20 bis 50 (m in Formel I ist dann 20 bis 50). Die Alkoxylierung kann auch mit einem Mischgas, z.B. aus Propylenoxid und Äthylenoxid, vorgenommen werden. Die Alkoxylierung der Glycerinäther erfolgt in der Regel in Gegenwart alkalischer Katalysatoren bei Temperaturen oberhalb 100°, z.B. in dem Temperaturbereich von 110 bis 130°C. Sie kann jedoch auch in Gegenwart saurer Katalysatoren erfolgen, z.B. Lewissäuren, wir $BF_3$—Ätherate.

Während alkoxylierte 1,3-Bisalkylglycerinäther ausgesprochene Tenside sind, handelt es sich bei den erfindungsgemäßen alkoxylierten 1,3-Bisarylglycerinäthern um wirksame Emulgatoren. Die neuen Stoffe können beispielsweise zum Emulgieren von Benzin, Weißöl, Phthalsäuredibutylester, Ricinusöl, Heptan, Toluol, Perchloräthylen oder veräthertes Methylolmelamin eingesetzt werden. Darüber hinaus besitzen die neuen Stoffe die Eigenschaft, feste, in Wasser unlösliche Teilchen, wie Pigmente und Farbstoffe, in feiner Verteilung zu halten. Sie verhindern das Agglomerieren bzw. Ausflocken der Teilchen.

Besonders wichtig ist die Verwendung der neuen Stoffe als Emulgator zur Herstellung von Druckpasten, und zwar besonders im benzinfreien und benzinarmen Pigmentdruck. Die neuen Stoffe können auch als Emulgator zur Herstellung von solchen Druckpasten verwendet werden, die auf Öl-in-Wasser-Emulsionen beruhen. Die Ölphase der Druckpasten wird hauptsächlich von Benzinfraktionen gebildet die in dem Bereich zwischen 80 und 250°C sieden. Man kann jedoch auch reine Kohlenwasserstoffe, die Hexane, Heptane, Nonane, Cyclohexylbenzole, Benzol, Toluole oder Xylole als Ölphase für die Druckpasten verwenden. Ebenso eignen sich Mischungen von Kohlenwasserstoffen. Die Druckpasten enthalten außer Wasser und dem organischen Lösungsmittel als weiteren Bestandteil Verdickungsmittel. Hierbei handelt es sich meist um wasserlösliche oder in Wasser quellbare Verdickungsmittel, wie Alginate, Johannisbrotkernmehläther, Stärkeäther oder Carboxymethylcellulose. Besonders wirksame Verbindungen sind z.B. synthetische Verdicker, wie die aus der DE—OS 20 54 885 bekannten. Diese Verdicker leiten sich von Di- und Polyurethanen von Polyätherdiolen ab. Sie sind in der genannten Offenlegungsschrift detailliert beschrieben und können auch zusammen mit den erfindungsgemäßen Emulgatoren zur Herstellung der Druckpasten werwendet werden. Je nach der gewünschten Viskosität der Druckpasten enthalten diese etwa 0,02 bis 1 Gew.%, vorzugsweise 0,05 bis 0,5 Gew.%, der genannten synthetischen Verdickungsmittel, bezogen auf die fertige Druckpaste. Die natürlichen Verdickungsmittel werden in höheren Mengen eingesetzt, z.B. bis zu 8 %.

Die Druckpasten enthalten außerdem im allgemeinen die für den Textildruck gebräuchlichen Bindemittel, z.B. diejenigen, die aus der DE—PS 1 140 898 bekannt sind. Für den Aufbau des Bindemittels kommen vorzugsweise Monomere in Betracht, die bei Raumtemperatur weiche und elastische Mischpolymerisate liefern, z.B. Vinylester höherer Carbonsäuren, wie Vinylpropionat, Acryl- und Methacrylsäureester, sowie Butadien oder seine Homologen. Die Monomeren werden entweder für sich allein

oder in Mischungen untereinander polymerisiert oder noch mit weiteren Monomeren mischpolymerisiert, beispielsweise mit Maleinsäure- und Fumarsäureester, Vinyläther, Vinylketon, Styrol, Vinylchlorid, Vinylidenchlorid, Vinylacetat, Acrylnitril und Methacrylsäuremethylester. Dazu können geringe Mengen wasserlöslicher polymerisierbarer Verbindungen, wie Acrylsäure, Vinylpyrrolidon, Amide ungesättigter Säuren oder die N-Methylolverbindungen bzw. N-Methyloläther dieser Amide einpolymerisiert werden. Die Bindemittel können noch andere Comonomere enthalten, die mindestens eine polymerisationsfähige Doppelbindung aufweisen, z.B. Ester, $\alpha,\beta$-äthylenisch ungesättigter organischer Säuren wie Acrylsäure oder Methacrylsäure mit höheren Alkoholen, welche in $\beta$-Stellung zu einer freien Hydroxylgruppe ein Halogenatom besitzen, z.B. Di-3-chlorpropandiol-1,2; 2,3-Dichlorbutandiol-1,4; oder 3-Chlor-2-chlormethyl-propandiol-1,2.

Bei der Herstellung der Öl-in-Wasser-Emulsionen können die für die Druckpastenherstellung üblichen Hilfsmittel verwendet werden, z.B. Entschäumer, pH-Wert-Regulatoren, Weichmacher und wasserlösliche Vorkondensate. Entschäumer werden üblicherweise in Mengen von 0,01 bis 0,15 Gew.%, bezogen auf die fertige Druckpaste eingesetzt. Geeignete Entschäumer sind beispielsweise Blockcopolymerisate des Äthylenoxids und/oder 1,2-Propylenoxids, die mit mehrwertigen Alkoholen veräthert und außerdem mit Carbonsäuren verestert sind. Derartige Produkte sind beispielsweise aus der DE—AS 21 14 609 bekannt.

Die fertige Druckpaste erhält man in an sich bekannter Weise, indem man beispielsweise die Emulsionsverdickung, die neben Wasser, Lösungsmittel, den erfindungsgemäßen Emulgator, und ggf. einen Entschäumer enthält, mit einem Pigmentfarbstoff bzw. einer Pigmentzubereitung, einem geeigneten Bindemittel und ggf. einem zusätzlichen wasserlöslichen Verdickungsmittel und weiteren in der Praxis üblichen Druckpastenbestandteilen zusammenrührt. Es ist jedoch auch möglich, daß ein Teil der Druckpastenbestandteile schon in der wäßrigen Emulgatorlösung vor dem Einemulgieren des Lösungsmittels anwesend ist. Einer zu dünnflüssig geratenen Druckpaste kann das Verdickungsmittel auch nachträglich zugesetzt werden. Eine Druckpaste kann so hergestellt werden, daß man zuerst eine Verdickeremulsion bereitet, indem man den Emulgator, ein Emulsionsverdickungsmittel, Wasser und Schwerbenzin, in dem ggf. ein Entschäumer gelöst ist, unter starkem Rühren vermischt.

Eine fertige Pigmentdruckpaste für den Rouleauxdruck erhält man beispielsweise durch Zusammenrühren von 60 Teilen einer Pigmentfarbstoffzubereitung (300 % Farbpigment), 790 Teilen der oben beschriebenen Verdickeremulsion, 120 Teilen eines Pigmentbinders auf der Basis eines der oben bezeichneten Copolymerisate in Form einer wäßrigen Dispersion und ggf. 10 bis 40 Teilen einer 33 %-igen wäßrigen Lösung von Diamoniumhydrogenphosphat als Säurespender. 1000 Gewichtsteile Druckpaste enthalten in der Regel 0,1 bis 40 Gewichtsteile Pigmentfarbstoff, 30 bis 100 Gewichtsteile eines 100 %-igen Bindemittels und 150 bis 650 Gewichtsteile eines Kohlenwasserstofföls, als wesentliche Bestandteile.

Die neuen Emulgatoren ermöglichen die Herstellung sehr stabiler Druckpasten. Mit diesen Druckpasten hergestellte Drucke sind sehr egal und brillant. Der Stand der Drucke ist sowohl im Filmdruck als auch im Rouleauxdruck ausgezeichnet. Die Druckpasten sind besonders auch für den Rotationsfilmdruck geeignet. Als Vorteil ist anzusehen, daß neben den geringen Mengen an Emulsionsverdickungsmitteln, wie sie beispielsweise in der DE—OS 20 54 885 beschrieben sind, die Druckpasten auch während der Herstellung schaumarm sowie lager- und scherstabil sind. Die mit den neuen Emulgatoren hergestellten Druckpasten können hauptsächlich für den Pigmentdruck auf Baumwolle, Zellwolle, Wolle, Glasfasergewebe oder anderen natürlichen und synthetischen Fasern sowie den verschiedensten Mischgeweben Verwendung finden. Es ist aber auch möglich, die Druckpasten z.B. mit herkömmlichen Farbstoffen, wie Dispersionsfarbstoffen oder Reaktivfarbstoffen, herzustellen.

Die Erfindung wird anhand der folgenden Beispiele näher erläutert. Die in den Beispielen genannten Teile und Prozente beziehen sich auf das Gewicht der Stoffe.

## Beispiel 1

Herstellung der alkoxylierten 1,3-Bisarylglycerinäther

610 Teile (5 Mol) 2,5-Dimethylphenol und 300 cm$^3$ Dioxan werden unter Rühren auf eine Temperatur in dem Bereich zwischen 50 und 60°C erwärmt und bei dieser Temperatur mit 110 Teilen (2,75 Mol) Natriumhydroxidpulver versetzt. Die Temperatur wird dann auf 90 bis 95°C erhöht. Das Gemisch wird 2 Stunden bei dieser Temperatur gerührt. Dann läßt man 231 Teile (2,5 Mol) Epichlorhydrin in Verlauf von 30 Minuten zulaufen und erhitzt die Mischung anschließend 4 Stunden unter Rückfluß. Das ausgefallene Kochsalz wird abfiltriert und das Dioxan in Vakuum abdestilliert. Man erhält 688 Gewichsteile 1,3-Bis-(2,5-dimethylphenyl)glycerinäther.

986 Teile (3,29 Mol) 1,3-Bis-(2,5-dimethylphenyl)glycerinäther werden mit 8 Teilen Kaliumhydroxid versetzt und in einem Rührautoklaven mit 2600 Teilen (59 Mol) Äthylenoxid, das portionsweise zugegeben wird, bei Temperaturen in dem Bereich von 110 bis 120°C zur Reaktion gebracht. Der Druck beträgt zwischen 4 und 9 bar. Man erhält 3590 Teile einer schwach gelben pastösen Substanz mit ausgezeichneten Emulgatoreigenschaften.

Nach der oben beschriebenen Verfahrensweise wurden die in der Tabelle angegebenen Phenole R—OH und R$^1$—OH im Molverhältnis 1:1:1 mit Epichlorhydrin umgesetzt. Der Äthoxylierungsgrad m ist ebenfalls in der Tabelle angegeben. Diese Zahl besagt, daß 1 Mol des Hydroxylgruppen enthaltenden

1,3-Bisarylglycerinäthers mit der m-fachen Molmenge an Äthylenoxid umgesetzt wurde. Im Gegensatz zu den aus der DE—PS 2 128 904 bekannten Emulgatoren können bei der Herstellung der erfindungsgemäßen Stoffe auch Isomerengemische, z.B. isomere Xylenole, eingesetzt werden, ohne die Wirksamkeit der alkoxylierten Produkte als Emulgator zu vermindern. Während sich der Alkylenoxidgehalt bei den bekannten Emulgatoren nur in einem sehr engen Bereich bewegte, kann dieser bei den neuen Stoffen in einem wesentlich breiteren Bereich schwanken, z.B. in dem Bereich von vorzugsweise 14 bis 30 Äthylenoxideinheiten.

Tabelle

| Bsp. 1 | R—OH | $R^1$—OH | m |
|---|---|---|---|
| a) | Phenol | $R^1 = R$ | 10 |
| b) | o-Kresol | $R^1 = R$ | 16 |
| c) | m-Kresol | $R^1 = R$ | 18 |
| d) | p-Kresol | $R^1 = R$ | 22 |
| e) | 4-Äthylphenol | $R^1 = R$ | 20 |
| f) | 2-Isopropylphenol | $R^1 = R$ | 25 |
| g) | 2,3-Xylenol | $R^1 = R$ | 16 |
| h) | 2,4-Xylenol | $R^1 = R$ | 18 |
| i) | 2,5-Xylenol | $R^1 = R$ | 22 |
| j) | 2,6-Xylenol | $R^1 = R$ | 20 |
| k) | 3,4-Xylenol | $R^1 = R$ | 20 |
| l) | 3,5-Xylenol | $R^1 = R$ | 18 |
| m) | 2,3,6-Trimethylphenol | $R^1 = R$ | 24 |
| n) | p-tert.-Butylphenol | $R^1 = R$ | 16 |
| o) | 2,4-Di-tert.-butylphenol | $R^1 = R$ | 22 |
| p) | Octylphenol | $R^1 = R$ | 18 |
| q) | Nonylphenol | $R^1 = R$ | 26 |
| r) | Nonylphenol | p-tert.-Butylphenol | 18 |
| s) | Nonylphenol | Phenol | 26 |
| t) | 2,4-Di-tert.-butylphenol | Phenol | 20 |
| u) | $\alpha$-Phenyläthylphenol | $R^1 = R$ | 20 |
| v) | Bis($\alpha$-Phenyläthyl)phenol | $R^1 = R$ | 30 |
| w) | Tris($\alpha$-Phenyläthyl)phenol | $R^1 = R$ | 50 |
| x) | $\alpha$-Naphthol | $R^1 = R$ | 45 |
| y) | $\beta$-Naphthol | $R^1 = R$ | 50 |

Herstellung der Druckpasten

Beispiel 2

140 Teile Wasser werden zusammen mit 50 Teilen einer 5 %-igen wäßrigen Lösung von Natriumalginat und 10 Teilen des im Beispiel 1 beschriebenen äthoxylierten 1,3-Bis(2,5-dimethyl-phenyl)-glycerinäthers vermischt. Dieser Mischung fügt man unter Rühren (etwa 3000 UpM) langsam 650 Teile Benzin und anschließend 150 Teile einer 40 %-igen wäßrigen Dispersion eines Copolymerisates aus 60 % n-Butylacrylat, 25 % Styrol, 10 % Acrylnitril, 4 % N-Methylolacrylamid und 1 % Acrylsäure zu. Man erhält eine viskose sehr stabile Öl-in-Wasser-Emulsion als Basis für Pigmentdruckfarben.

Eine Mischung aus 960 Teilen der Öl-in-Wasser-Emulsion wird mit 40 Teilen einer handelsüblichen wäßrigen, etwa 40%-igen Pigmentzubereitung %Phthalocyaninanteigung) vermischt. Man erhält eine sehr stabile Druckpaste, die auch höchsten Ansprüchen genügt. Mit der so hergestellten Druckpaste wird ein Zellwollgewebe im Rouleauxdruck bedruckt. Man erhält einen Druck von besonders hoher Farbstoffausbeute und guter Brillanz.

Beispiel 3

40,0 Teile eines ca. 30%-igen wäßrigen Farbstoffteiges (C.I. 71103), 115,0 Teile einer handels--üblichen 6%-igen wäßrigen Lösung von polyacrylsaurem Ammonium, 120,0 Teile einer 40%-igen wäßrigen Dispersion eines Copolymerisates aus 80% n-Butylacrylat, 15% Acrylnitril, 4.5% N-Methylolacrylamid und 0,5% Acrylsäure, 10,0 Teile des im Beispiel 1 angegebenen äthoxylierten 1,3-Bisarylglycerinäthers als Emulgator, 20,0 Teile Harnstofflösung 50 %-ig, 0,5 Teile Triäthanolamin, 3,0 Teile Glycerin, 30,0 Teile Stearinsäure-i-decylester, 661,5 Teile Wasser werden zusammengegeben und unter Rühren homogenisiert. Man erhält so eine stabile, von störenden Agglomeraten freie, sehr gut verarbeitare benzinfreie Druckpaste.

Mit dieser Druckpaste bedruckt man in bekannter Weise auf einer üblichen Rotationsfilmdruckmaschine ein Baumwollgewebe. Es resultieren farbtiefe und brillante Drucke.

Beispiel 4

Man stellt zunächst eine Halbemulsion aus folgenden Bestandteilen her:

| | |
|---|---|
| Wasser | 165 g |
| Emulgator gemäß Beispiel 2 | 5 g |
| Alginatverdickung 5 %-ig | 400 g |
| Benzin | 430 g |
| | 1000 g |

Zu 600 Teilen dieser Emulsion gibt man dann 270 Teile Wasser, 100 Teile Harnstoff, 10 Teile 3-nitrobenzolsulfonsaures Natrium und 20 Teile Soda. Man erhält eine als Stammverdikkung bezeichnete Mischung, aus der durch Zugabe von Farbstoffen die fertigen Druckpasten resultieren. Jeweils 970 Teile der Stammverdickung wurden 1 × mit dem Reaktivfarbstoff der C.I. Nr. 13 245 und zum anderen mit 30 g des gelben Reaktivfarbstoffs der C.I. Nr. 61 205:1 gemischt. In beiden Fällen erhielt man stabile Druckpasten, die im Filmdruck auf Baumwolle gedruckt wurden. Man erheilt jeweils farbtiefe, leuchtende, brillante Drucke.

Beispiel 5

Es wurde zunächst eine Emulsion hergestellt, in dem man 400 Teile einer 8%-igen wäßrigen Kernmehlätherverdickung, 330 Teile Wasser, 10 Teile des Emulgators gemäß Beispiel 1, und 10 Teile 3-nitrobenzolsulfonsaures Natrium miteinander vermischte. Dann emulgiert man 250 Teile Benzin in diese Mischung ein. Durch Zugabe von Zitronensäure wurde ein pH-Wert von 5,5 eingestellt. 960 Teile der Emulsion wurden anschließend mit 40 Teilen des Dispersionsfarbstoffs der C.I. Nr. 62 030 gemischt.

Es wurde eine weitere Druckpaste hergestellt, indem man 960 Teile der oben beschriebenen Emulsion mit 40 Teilen des Dispersionsfarbstoffs der C.I. Nr. 60 756 mischte. Die Druckpasten wurden im Filmdruck auf Polyester-Satin gedruckt. Das bedruckte Material wurde 6 Minuten bei einer Temperatur von 175°C mit Heißdampf fixiert und in üblicher Weise nachgewaschen. Man erhielt in beiden Fällen einen Druck auf Polyester-Satin, der sich durch Farbtiefe, gute Farbausbeute und Stippenfreiheit auszeichnete.

**Patentansprüche**

1. Alkoxylierte 1,3-Bisarylglycerinäther der Formel

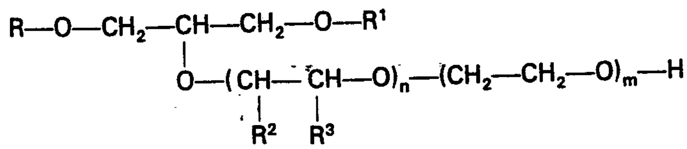

(I)

in der

R, $R^1$ = 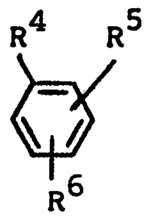 , α-Naphthyl oder β-Naphthyl und

$R^4$, $R^5$, $R^6$ = —H, eine $C_1$- bis $C_{12}$-Alkylgruppe oder

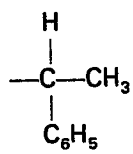

$R^2$ = $-CH_3$, $-C_2H_5$,
$R^3$ = —H, $-CH_3$,
n = 0 bis 10 und
m = 8 bis 50
bedeuten.

2. Verwendung der alkoxylierten Glycerinäther nach Anspruch 1 als Emulgator.

3. Verwendung der alkoxylierten Glycerinäther nach Anspruch 1 als Emulgator zur Herstellung von Druckpasten in einer Menge von 0,1 bis 1,5 Gew.%, bezogen auf die Druckpasten.

**Claims**

1. An oxyalkylated 1,3-bis-aryl-glycerol ether of the formula

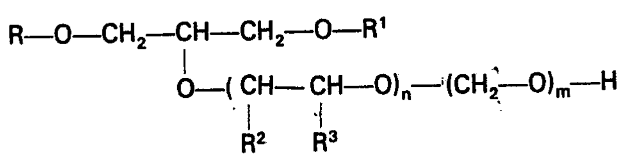

(I)

where R and $R^1$ are 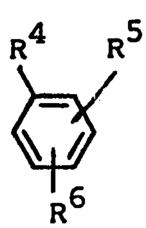 , α-naphthyl or β-naphthyl

and $R^4$, $R^5$ and $R^6$ are —H, $C_1$—$C_{12}$-alkyl or

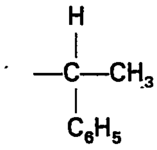

$R^2$ is —$CH_3$ or —$C_2H_5$, $R^3$ is —H or —$CH_3$, n is from 0 to 10 and m is from 8 to 50.

2. Use of an oxyalkylated glycerol ether as claimed in claim 1 as emulsifier.

3. Use of an oxyalkylated glycerol ether as claimed in claim 1 as emulsifier for the production of print pastes, in an amount of from 0.1 to 1.5% by weight, based on the print paste.

**Revendications**

1. Ether 1,3-bisarylglycérique alcoxylé, de formule

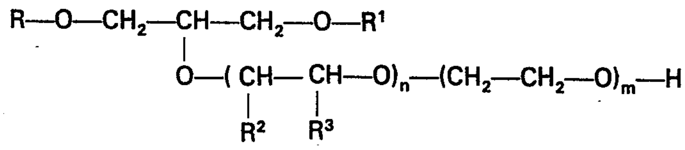

(I)

dans laquelle

R, $R^1$ = 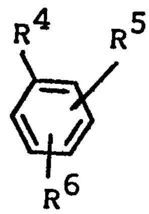 , $\alpha$-naphtyle ou $\beta$-naphtyle et

$R^4$, $R^5$, $R^6$ = —H, un groupe alkyle en $C_1$ à $C_{12}$ ou

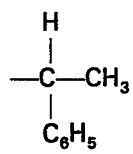

$R^2$ = —$CH_3$, —$C_2H_5$,
$R^3$ = —H, —$CH_3$,
n = 0 à 10 et
m = 8 à 50

2. Utilisation de l'éther glycérique alcoxylé selon la revendication 1, comme émulsifiant.

3. Utilisation de l'éther glycérique alcoxylé selon la revendication 1, comme émulsifiant pour la préparation de pâtes d'impression, en proportion de 0,1 à 1.5 % en poids, rapportée aux pâtes d'impression.